# EUROPEAN PATENT SPECIFICATION

(11) **EP 3 719 178 B1**
(45) Date of publication and mention of the grant of the patent: **16.03.2022**
(21) Application number: 19209251.8
(22) Date of filing: 14.11.2019
(51) Int. Cl.: C25B 1/13, C25B 1/24, C25B 15/02, A62C 3/06, A62C 3/08, A62C 99/00

(54) **ELECTROCHEMICAL ANTI-MICROBIAL TREATMENT AND INERT GAS GENERATING SYSTEM AND METHOD**
ELEKTROCHEMISCHES SYSTEM UND VERFAHREN ZUR ANTIMIKROBIELLEN BEHANDLUNG UND INERTGASERZEUGUNG
SYSTÈME ET PROCÉDÉ ÉLECTROCHIMIQUES POUR TRAITEMENT ANTIMICROBIEN ET GÉNÉRATION DE GAZ INERTE

(30) Priority: 04.04.2019 US 201916375609
(43) Date of publication of application: 07.10.2020
(73) Proprietor: Hamilton Sundstrand Corporation, Charlotte, NC 28217-4578 (US)
(72) Inventor: PESS, Matthew, Hartford, CT 06107 (US); RHEAUME, Jonathan, West Hartford, CT 06119 (US); PERRY, Michael L., Glastonbury, CT 06033 (US)
(74) Representative: Dehns

(56) References cited:
- CN-A- 109 321 937
- US-A1- 2018 001 124
- US-A1- 2019 001 264

## Description

### BACKGROUND

The subject matter disclosed herein generally relates to vehicles comprising on-board systems for generating and providing inert gas to protected spaces and to providing anti-microbial treatment as well, and to corresponding methods.

It is recognized that fuel vapors within fuel tanks become combustible or explosive in the presence of oxygen. An inerting system decreases the probability of combustion or explosion of flammable materials in a fuel tank by maintaining a chemically non-reactive or inerting gas, such as nitrogen-enriched air, in the fuel tank vapor space, also known as ullage. Three elements are required to initiate combustion or an explosion: an ignition source (e.g., heat), fuel, and oxygen. The oxidation of fuel may be prevented by reducing any one of these three elements. If the presence of an ignition source cannot be prevented within a fuel tank, then the tank may be made inert by: 1) reducing the oxygen concentration, 2) reducing the fuel concentration of the ullage to below the lower explosive limit (LEL), or 3) increasing the fuel concentration to above the upper explosive limit (UEL). Many systems reduce the risk of oxidation of fuel by reducing the oxygen concentration by introducing an inerting gas such as nitrogen-enriched air (NEA) (i.e., oxygen-depleted air or ODA) to the ullage, thereby displacing oxygen with a mixture of nitrogen and oxygen at target thresholds for avoiding explosion or combustion. CN 109321937 A discloses an electrolytic ozone generator comprising an electrochemical cell, a cathode fluid chamber having a an air inlet, an anode fluid chamber with a water inlet, and an outlet for water and ozone.

It is known in the art to equip vehicles (e.g., aircraft, military vehicles, etc.) with onboard inerting gas generating systems, which supply nitrogen-enriched air to the vapor space (i.e., ullage) within the fuel tank. The nitrogen-enriched air has a substantially reduced oxygen content that reduces or eliminates oxidizing conditions within the fuel tank. Onboard inerting gas generating systems typically use membrane-based gas separators. Such separators contain a membrane that is permeable to oxygen and water molecules, but relatively impermeable to nitrogen molecules. A pressure differential across the membrane causes oxygen molecules from air on one side of the membrane to pass through the membrane, which forms oxygen-enriched air (OEA) on the low-pressure side of the membrane and nitrogen-enriched air (NEA) on the high-pressure side of the membrane. The requirement for a pressure differential necessitates a source of compressed or pressurized air. Another type of gas separator is based on an electrochemical cell such as a proton exchange membrane (PEM) electrochemical cell, which produces NEA by electrochemically generating protons for combination with oxygen to remove it from air.

Additionally, protected spaces such as fuel tanks can be susceptible to microbial contamination, and other systems associated with or in proximity to protected spaces can also be susceptible to microbial contamination, including but not limited to water storage systems such as aircraft on-board water systems, which can be used to provide water for lavatory and other on-board facilities and for which microbial contamination can constitute a health risk.

Accordingly, such on-board systems require substantial maintenance when the system is off-line to maintain safety and quality, and dedicated treatment systems such as chlorination or reverse osmosis systems can add additional payload, which in turn increases aircraft operating costs such as fuel consumption. As a result, many systems such as water supply systems or fuel systems can be susceptible to microbial contamination.

### BRIEF DESCRIPTION

In an aspect, vehicle having an inert gas-generating system according to claim 1 is provided.

In some aspects, the ozone flow path can include a gas-liquid separator that receives a mixture comprising process water, oxygen, and ozone from the anode fluid flow path outlet and outputs a gas comprising ozone to the ozone storage or distribution system.

In any one or combination of the foregoing aspects, the vehicle can further comprise a biologically active surface or material, wherein the ozone storage or distribution system can be in controllable operative fluid communication with the biologically active surface or material.

In any one or combination of the foregoing aspects, the biologically active surface or material can include a water storage tank, or a water distribution system, or a fuel storage tank, or a fuel distribution system.

[deleted]

[deleted]

In any one or combination of the foregoing aspects, the ozone storage or distribution system can be in controllable operative fluid communication with a liquid space or a vapor space of a water storage or supply tank.

In any one or combination of the foregoing aspects, the ozone storage or distribution system can be in controllable operative fluid communication with a water supply flow path.

In any one or combination of the foregoing aspects, the vehicle can further include a controller configured to direct a gas comprising ozone to the gas-liquid contactor in response to a flow of water on the water supply flow through the gas-liquid contactor.

[deleted]

In any one or combination of the foregoing aspects, the controller can be configured to operate in the first mode continuously or at intervals under normal operating conditions, and to operate in the second mode in response to a demand for emergency electrical power.

Also disclosed is a method of treating a biologically active surface or material according to claim 7.

In any one or combination of the foregoing aspects, the method can further include directing a fluid from the anode fluid flow path outlet to a gas-liquid separator, and directing the gas mixture comprising ozone from the cathode fluid flow path outlet and outputs a gas comprising ozone to the ozone storage or distribution system.

In any one or combination of the foregoing aspects, the method can further include operating the electrochemical cell and directing the gas comprising ozone to the gas-liquid separator in response to a flow of water on the aircraft water supply flow through the gas-liquid separator.

In any one or combination of the foregoing aspects, the biologically active surface or material can include a water storage tank, or a water distribution system, or a fuel storage tank, or a fuel distribution system.

In any one or combination of the foregoing aspects, the biologically active surface or material can include a water storage tank, and the method includes sparging the gas comprising ozone through a liquid space in the water storage tank.

In any one or combination of the foregoing aspects, the biologically active surface or material can include a water distribution system, and the method includes contacting gas flowing through the water distribution system with a stream of the gas comprising ozone.

In any one or combination of the foregoing aspects, the biologically active surface or material can include a fuel storage tank or a fuel distribution system, and the method includes inerting the fuel storage tank or fuel distribution system, and adding the gas comprising ozone to the fuel tank or fuel distribution system.

In any one or combination of the foregoing aspects, inerting the fuel storage tank or distribution system includes adding an inert gas to the fuel tank or fuel distribution system.

In any one or combination of the foregoing aspects, the method can further include operating in alternate modes of operation selected from a plurality of modes including: (i) a first mode in which process water is directed to the anode fluid flow path inlet, electric power is directed from the power source to the electrochemical cell to provide a voltage difference between the anode and the cathode, and a gas comprising ozone is directed from the anode fluid flow path outlet to the ozone storage or distribution system; and (ii) a second mode in which hydrogen is directed from the hydrogen source to the anode fluid flow path inlet, electric power is directed from the electrochemical cell to the power sink, and the ozone storage or distribution system is isolated from the anode fluid flow path outlet.

### BRIEF DESCRIPTION OF THE DRAWINGS

The following descriptions should not be considered limiting in any way. With reference to the accompanying drawings, like elements are numbered alike:
FIG. 1A is a schematic illustration of an aircraft that can incorporate various embodiments of the present disclosure;
FIG. 1B is a schematic illustration of a bay section of the aircraft of FIG. 1A;
FIG. 2 is a schematic depiction an example embodiment of an electrochemical cell;
FIG. 3 is a schematic illustration of an example embodiment of an electrochemical inerting and treatment system;
FIG. 4 is a schematic illustration of an example embodiment of ozone storage or distribution; and
FIG. 5 is a schematic illustration of another example embodiment of ozone storage or distribution.

### DETAILED DESCRIPTION

A detailed description of one or more embodiments of the disclosed apparatus and method are presented herein by way of exemplification and not limitation with reference to the Figures.

Although shown and described above and below with respect to an aircraft, embodiments of the present disclosure are applicable to on-board systems for any type of vehicle . . For example, military vehicles, heavy machinery vehicles, sea craft, ships, submarines, etc., may benefit from implementation of embodiments of the present disclosure. For example, aircraft and other vehicles having fire suppression systems, emergency power systems, and other systems that may electrochemical systems as described herein may include the redundant systems described herein. As such, the present disclosure is not limited to application to aircraft, but rather aircraft are illustrated and described as example and explanatory embodiments for implementation of embodiments of the present disclosure.

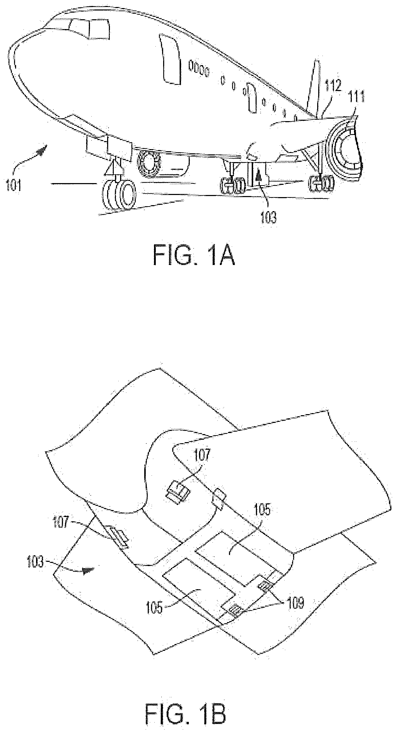

As shown in FIGS. 1A-1B, an aircraft includes an aircraft body 101, which can include one or more bays 103 beneath a center wing box. The bay 103 can contain and/or support one or more components of the aircraft 101. For example, in some configurations, the aircraft can include environmental control systems (ECS) and/or on-board inerting gas generation systems (OBIGGS) within the bay 103. As shown in FIG. 1B, the bay 103 includes bay doors 105 that enable installation and access to one or more components (e.g., OBIGGS, ECS, etc.). During operation of environmental control systems and/or fuel inerting systems of the aircraft, air that is external to the aircraft can flow into one or more ram air inlets 107. The outside air may then be directed to various system components (e.g., environmental conditioning system (ECS) heat exchangers) within the aircraft. Some air may be exhausted through one or more ram air exhaust outlets 109.

Also shown in FIG. 1A, the aircraft includes one or more engines 111. The engines 111 are typically mounted on the wings 112 of the aircraft and are connected to fuel tanks (not shown) in the wings, but may be located at other locations depending on the specific aircraft configuration. In some aircraft configurations, air can be bled from the engines 111 and supplied to OBIGGS, ECS, and/or other systems, as will be appreciated by those of skill in the art.

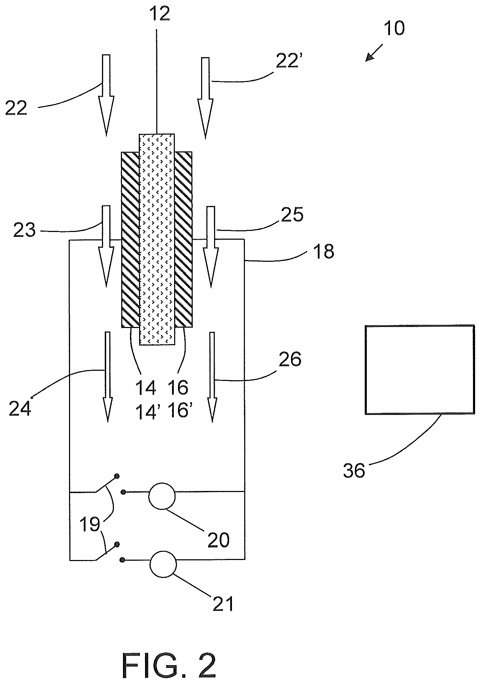

Referring now to FIG. 2, an electrochemical cell is schematically depicted. The electrochemical cell 10 comprises a separator 12 that includes an ion transfer medium. As shown in FIG. 2, the separator 12 has a cathode 14 disposed on one side and an anode 16 disposed on the other side. Cathode 14 and anode 16 can be fabricated from catalytic materials suitable for performing the needed electrochemical reaction (e.g., the oxygen-reduction reaction at the cathode and an oxidation reaction at the anode). Exemplary catalytic materials include, but are not limited to, nickel, platinum, palladium, rhodium, carbon, gold, tantalum, titanium, tungsten, ruthenium, iridium, osmium, zirconium, alloys thereof, and the like, as well as combinations of the foregoing materials. Cathode 14 and anode 16, including catalyst 14' and catalyst 16', are positioned adjacent to, and preferably in contact with the separator 12 and can be porous metal layers deposited (e.g., by vapor deposition) onto the separator 12, or can have structures comprising discrete catalytic particles adsorbed onto a porous substrate that is attached to the separator 12. Alternatively, the catalyst particles can be deposited on high surface area powder materials (e.g., graphite or porous carbons or metal-oxide particles) and then these supported catalysts may be deposited directly onto the separator 12 or onto a porous substrate that is attached to the separator 12. Adhesion of the catalytic particles onto a substrate may be by any method including, but not limited to, spraying, dipping, painting, imbibing, vapor depositing, combinations of the foregoing methods, and the like. Alternately, the catalytic particles may be deposited directly onto opposing sides of the separator 12. In either case, the cathode and anode layers 14 and 16 may also include a binder material, such as a polymer, especially one that also acts as an ionic conductor such as anion-conducting ionomers. In some embodiments, the cathode and anode layers 14 and 16 can be cast from an "ink," which is a suspension of supported (or unsupported) catalyst, binder (e.g., ionomer), and a solvent that can be in a solution (e.g., in water or a mixture of alcohol(s) and water) using printing processes such as screen printing or ink jet printing.

The cathode 14 and anode 16 are controllably electrically connected by electrical circuit 18 to a controllable electric power system 20, which includes a power source (e.g., DC power rectified from AC power produced by a generator powered by a gas turbine engine used for propulsion or by an auxiliary power unit) a power sink 21. The electric power system 20 includes a connection to the electric power sink 21 (e.g., one or more electricity-consuming systems or components onboard the vehicle) with appropriate switching (e.g., switches 19), power conditioning, or power bus(es) for such on-board electricity-consuming systems or components, for optional operation in an alternative fuel cell mode.

With continued reference to FIG. 2, a cathode supply fluid flow path 22 directs gas from an air source (not shown) into contact with the cathode 14. Oxygen is electrochemically depleted from air along the cathode fluid flow path 23, and can be exhausted to the atmosphere or discharged as nitrogen-enriched air (NEA) (i.e., oxygen-depleted air, ODA) to an inerting gas flow path 24 for delivery to an on-board fuel tank (not shown), or to a vehicle fire suppression system associated with an enclosed space (not shown), or controllably to either or both of a vehicle fuel tank or an on-board fire suppression system. An anode fluid flow path 25 is configured to controllably receive an anode supply fluid from an anode supply fluid flow path 22'. The anode fluid flow path 25 includes water when the electrochemical cell is operated in an electrolytic mode to produce protons at the anode for proton transfer across the separator 12 (e.g., a proton transfer medium such as a proton exchange membrane (PEM) electrolyte or phosphoric acid electrolyte). The anode fluid flow path 25 is configured to controllably also receive fuel (e.g., hydrogen). The protons formed at the anode are transported across the separator 12 to the cathode 14, leaving oxygen and ozone on the anode fluid flow path, which is exhausted through an anode exhaust 26. The formation of ozone can be promoted with an elevated cell voltage (e.g. 2.1-3 Volts). Catalysts can also be formulated to favor promotion of the ozone-forming reaction. For example, the platinum-group metals (e.g., platinum, palladium, rhodium, iridium, rhuthenium, osmium) can produce ozone at the anode, and other catalysts can produce ozone at higher efficiencies, e.g., glassy carbon (e.g., boron-doped diamond), or metal oxide catalysts such as PbO₂, Ta₂O₅. It is likely that both ozone (O₃) and diatomic oxygen (O₂) will be generated simultaneously, and that the ozone produced will be mixed in with oxygen and any process water beyond that needed for stoichiometric operation. Control of fluid flow along these flow paths can be provided through conduits and valves (not shown), which can be controlled by a controller 36. The controller can include a microprocessor that is programmed with instructions for sending signals to carry out control of any of the operations described herein.

Exemplary materials from which the electrochemical proton transfer medium can be fabricated include proton-conducting ionomers and ion-exchange resins. Ion-exchange resins useful as proton conducting materials include hydrocarbon- and fluorocarbon-type resins. Fluorocarbon-type resins typically exhibit excellent resistance to oxidation by halogen, strong acids, and bases. One family of fluorocarbon-type resins having sulfonic acid group functionality is NAFION^{™} resins (commercially available from E. I. du Pont de Nemours and Company, Wilmington, Del.). Alternatively, instead of an ion-exchange membrane, the separator 12 can be comprised of a liquid electrolyte, such as sulfuric or phosphoric acid, which may preferentially be absorbed in a porous-solid matrix material such as a layer of silicon carbide or a polymer than can absorb the liquid electrolyte, such as poly(benzoxazole). These types of alternative "membrane electrolytes" are well known and have been used in other electrochemical cells, such as phosphoric-acid fuel cells.

During operation of a proton transfer electrochemical cell in the electrolytic mode, water at the anode undergoes an electrolysis reaction according to the formulae:

H₂O → ½O₂ + 2H⁺ + 2e⁻ (1a)

3H₂O → O₃ + 6H⁺ + 6e⁻ (1b)

The electrons produced by this reaction are drawn from electrical circuit 18 powered by electric power source 20 connecting the positively charged anode 16 with the cathode 14. The hydrogen ions (i.e., protons) produced by this reaction migrate across the separator 12, where they react at the cathode 14 with oxygen in the cathode flow path 23 to produce water according to the formula

½O₂ + 2H⁺ + 2e⁻ → H₂O (2)

Removal of oxygen from cathode flow path 23 produces nitrogen-enriched air exiting the region of the cathode 14. The oxygen and ozone evolved at the anode 16 by the reaction of formula (1) is discharged as anode exhaust 26.

During operation of a proton transfer electrochemical cell in a fuel cell mode, fuel (e.g., hydrogen) at the anode undergoes an electrochemical oxidation according to the formula

H₂ → 2H⁺ + 2e (3)

The electrons produced by this reaction flow through electrical circuit 18 to provide electric power to the electric power sink 21. The hydrogen ions (i.e., protons) produced by this reaction migrate across the separator 12, where they react at the cathode 14 with oxygen in the cathode flow path 23 to produce water according to the formula (2).

½O₂ + 2H⁺ + 2e⁻ → H₂O (2)

Removal of oxygen from cathode flow path 23 produces nitrogen-enriched air exiting the region of the cathode 14.

As mentioned above, the electrolysis reaction occurring at the positively charged anode 16 requires water, and the ionic polymers used for a PEM electrolyte perform more effectively in the presence of water. Accordingly, in some embodiments, a PEM membrane electrolyte is saturated with water or water vapor. Although the reactions (1a-b) and (2) are stoichiometrically balanced with respect to water so that there is no net consumption of water, in practice some amount of moisture will be removed through the cathode exhaust 24 and/or the anode exhaust 26 (either entrained or evaporated into the exiting gas streams). Accordingly, in some exemplary embodiments, water from a water source is circulated past the anode 16 along an anode fluid flow path (and optionally also past the cathode 14). Such water circulation can also provide cooling for the electrochemical cells. In some exemplary embodiments, water can be provided at the anode from humidity in air along an anode fluid flow path in fluid communication with the anode. In other embodiments, the water produced at cathode 14 can be captured and recycled to anode 16 (e.g., through a water circulation loop, not shown). It should also be noted that, although the embodiments are contemplated where a single electrochemical cell is employed, in practice multiple electrochemical cells will be electrically connected in series with fluid flow to the multiple cathode and anode flow paths routed through manifold assemblies.

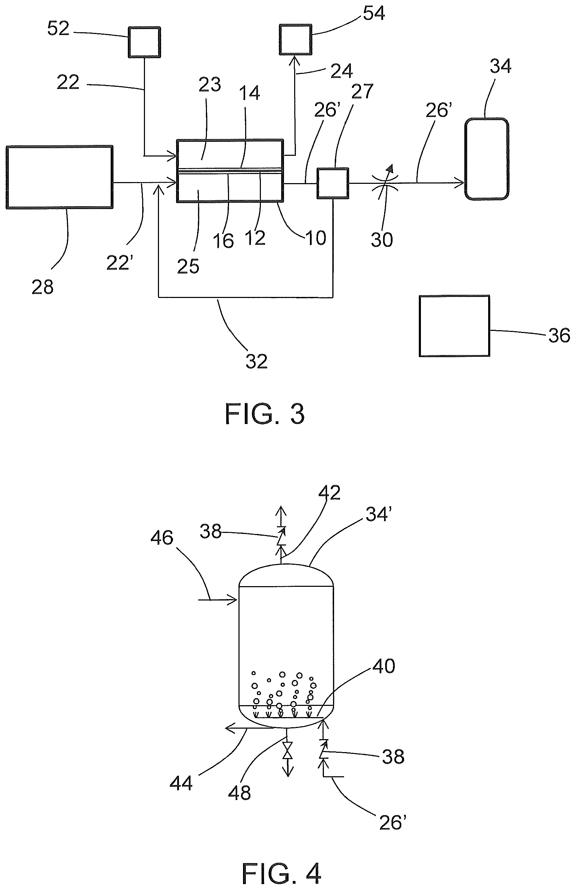

An example embodiment of an aircraft inert gas-generating system that produces ozone from an electrochemical cell 10 is schematically shown in FIG. 3. As shown in FIG. 3, water from a process water source 28 is directed (e.g., by a pump, not shown) along the anode supply fluid flow path 22' to the anode fluid flow path 25, where it is electrolyzed at the anode 16 to form protons, ozone, and oxygen. The protons are transported across the separator 12 to the cathode 14, where they combine with oxygen from airflow along the cathode fluid flow path 23 to form water. Removal of the protons from the anode fluid flow path 25 leaves ozone and oxygen gas on the anode fluid flow path, which is discharged as anode exhaust 26 to an ozone fluid flow path 26'.

As further shown in FIG. 3, the ozone fluid flow path 26' includes a gas-liquid separator 27 and a flow control valve 30. Although water is consumed at the anode by electrolysis, the gas exiting as anode exhaust 26 can include water vapor or entrained liquid water from excess water on the anode fluid flow path 25 such as from a liquid water circulation loop. The gas-liquid separator 27 can include a tank with a liquid space and a vapor space inside, allowing for liquid water to be removed from the liquid space and transported back to the electrochemical cell 10 through water return conduit 32. Heat may be provided to promote evolution of ozone gas from ozone dissolved in the process water. Additional gas-liquid separators and/or water removal devices can be used such as a coalescing filter, vortex gas-liquid separator, electrochemical dryer, or membrane separator, to remove moisture from the ozone if needed (e.g., moisture removal may be needed if the ozone is used to treat moisture-sensitive areas such as fuel tanks or fuel systems). Additional gas-liquid separators can include coalescing filters, vortex gas-liquid separators, or membrane separators, and can be located for example along the fluid flow path 26'. Examples of water removal devices include but are not limited to a desiccant (including a desiccant wheel), a membrane drier (see, e.g., US 2019/0001264A1), a condensing heat exchanger operated at elevated pressure (see, e.g., US patent application serial no. 16,149,736), or other water removal device (e.g., gas-liquid separators such as a coalescing filter, or vortex gas-liquid separator, or an electrochemical dryer, see, e.g., US patent application serial no. 16,127,980 and US20190001264A1), and can be used to remove water vapor and entrained liquid water.

As further shown in FIG. 3, the electrochemical cell or cell stack 10 generates an inerting gas on the cathode fluid flow path 23 by depleting oxygen to produce oxygen-depleted air (ODA), also known as nitrogen-enriched air (NEA) at the cathode 14 that is directed to a protected space 54 (e.g., a fuel tank ullage space, a cargo hold, or an equipment bay). As shown in FIG. 3, an air source 52 (e.g., ram air, compressor bleed, blower) is directed to the cathode fluid flow path 23 where oxygen is depleted by electrochemical reactions with protons that have crossed the separator 12 as well as electrons from an external circuit (not shown) to form water at the cathode 14. The ODA thereby produced is directed to a protected space 54 such as an ullage space in in the aircraft fuel tanks as disclosed or other protected space 54. The inerting gas flow path (cathode exhaust 24) can include additional components (not shown) such as flow control valve(s), a pressure regulator or other pressure control device, and water removal device(s) such as a heat exchanger condenser, a membrane drier or other water removal device(s), or a filter or other particulate or contaminant removal devices. Additional information regarding the electrochemical production of ODA can be found in U.S. Patent 9,963,792, US Patent Application Publication No. 2017/0331131A1, and US Patent Application Serial No. 16/029,024.

As further shown in FIG. 3, the gas comprising ozone on the ozone flow path 26' is delivered to an ozone storage or distribution system 34, where it can stored and/or distributed provide a biocidal effect for disinfecting or otherwise treating organic contaminants at a biologically-active material or surface such as a water supply tank, a water distribution system, grey-water holding tank, a fuel tank or a fuel distribution system. Ozone itself is a strong oxidant that can provide the biocidal effect, and can also decompose to form hydroxyl or peroxyl radicals that are also reactive with organic contaminants. Microbial contamination of water or fuel system components can lead to formation of a sludge-like biomass that can clog filters, occlude conduit lines, and lead to unplanned system failure. Acidic byproducts of metabolism of microbes can alter the pH of fuel or water tanks and conduits and promote corrosion or scale formation. In fuel systems, microbial growth can be promoted by the introduction of water vapor to a fuel tank often in the form of vapor through a vent. Aircraft fuel tanks can be subject to significant incoming moisture during descent, as moisture-containing outside air at a pressure greater than that of pressure in the fuel tank enters through one or more fuel system vents. By a similar pathway, microbes and spores can find their way into fuel tanks. The condensation of water vapor in the fuel tank causes the liquid water to come into contact with the fuel. Water and fuel are immiscible, so the water settles at the bottom of the tank, where the interface of the water and fuel can provide an environment for microbial growth involving fungus or bacteria, or both fungus and bacteria.

Additional detail regarding the storage or distribution of the gas comprising ozone is shown in an example embodiment of FIG. 4. As shown in FIG. 4, the gas comprising ozone and oxygen is received from the liquid-gas separator 27 (FIG. 3) on the ozone fluid flow path 26', and passes through a check valve 38, and then is dispensed into a tank 34'. The tank 34' can be an ozone storage tank, or it can be a material or surface to be treated with ozone such as a water supply tank or a fuel tank. In the case of an ozone storage tank, ozone is introduced to the tank through manifold 40 and dispensed through conduit 44. In the case of a water supply tank, the ozone can be delivered into a liquid water space in the tank 34' through a sparging manifold 40, and the bubbles of ozone contacting the water can promote a biocidal effect. A vent 42 with another check valve 38 allows for venting of pressure from gas buildup. Water can be dispensed from the storage tank 34 through conduit 44 to on-board water usage stations such as lavatory or galley facilities. A fill line 46 allows for initial charging of the water supply system with water, and a drain line 48 allows for draining of the system for cleaning and maintenance. In the case of treatment of a fuel tank or fuel system component, the tank or component can be inerted by establishing conditions such that addition of ozone will not form a combustible mixture. Inerting the tank or component can involve removal of fuel and/or adding inert gas to the fuel tank or component. Ozone gas can then be added, e.g., through the manifold 40, held in the tank or component for biocidal effect, and then vented through vent 42. In another embodiment, the ozone-containing anode effluent is introduced to a fuel tank that contains fuel and or water for in-situ sparging. In this case, it is envisioned to provide sufficient inert gas to the fuel tank ullage to avoid the formation of a combustible or explosive mixture of fuel and air. Additional protocols can be employed for cleaning of fuel tanks and systems, including but not limited to solvent cleaning to solubilize and remove lower volatility hydrocarbons, and purging a fuel tank or component with inert gas (such as produced at the cathode 14) and/or with air prior to or simultaneous with introduction of the ozone-containing gas to a fuel tank or fuel system component.

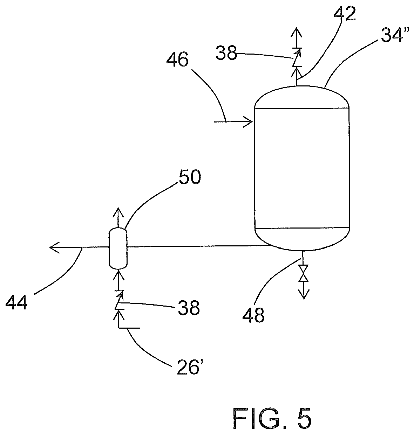

In another embodiment, instead of treating a water supply system by introducing ozone directly into a water supply tank such as tank 34', the gas comprising ozone can be introduced to a gas-liquid contactor 50 disposed along conduit 44 serving as a water supply line, as shown in the example embodiment of FIG. 5. This can provide a technical effect of promoting anti-microbial action only in water as it is being dispensed. However, ozone should not reach consumers of water from a treated water system, and in some embodiments, a UV light source (not shown) can be used to dissociate ozone. A UV light source can be located at a point of use (e.g., between an ozone point of contact such as gas-liquid contactor 50 and a water dispenser) or can be disposed either in the tank 34' or at outlets from the tank 34' if the ozone introduction point is the tank 34' and water residence time in the tank is not sufficient for ozone to dissociate on its own over time.

In some embodiments, the electrochemical cell 10 can be operated continuously for delivery of ozone to the ozone storage or distribution system 34. However, continuous operation may not be necessary to meet system needs, and in some embodiments, the electrochemical cell 10 can be operated at to produce ozone at regular or irregular intervals. For example, in some embodiments, the electrochemical cell 10 can be operated in response to a predetermined quantity of water passing through a water storage tank (i.e., a degree of tank turnover). In some embodiments, the electrochemical cell 10 can be operated in response to detection of water passing through conduit 44 as a water supply line or through the gas-liquid contactor 50. In some embodiments, the electrochemical cell can be operated in response to a predetermined period of time such as a timer operating in the processor of controller 36.

Although this disclosure includes embodiments where an electrochemical cell is utilized exclusively for producing ozone and inert gas, the electrochemical cell is also used for other purposes, namely the electrochemical cell is used to in an alternate mode to provide electric power for on-board power-consuming systems, as disclosed in the aforementioned US Patent Application Publication No. 2017/0331131A1. In this mode, fuel hydrogen) is directed from a fuel source to the anode 16 where hydrogen molecules are split to form protons that are transported across the separator 12 to combine with oxygen at the cathode. Simultaneously, reduction and oxidation reactions exchange electrons at the electrodes, thereby producing electricity in an external circuit. Ozone is not produced by the electrochemical cell in this mode, and the water supply system usually can go untreated for short periods such as during an electricity-production mode. Embodiments in which these alternate modes of operation can be utilized include, for example, operating the system in alternate modes selected from a plurality of modes including a first mode of electrochemical oxygen production under normal aircraft operating conditions (e.g., in which an engine-mounted generator provides electrical power) and a second mode of electrochemical electricity production (e.g., in response to a demand for emergency electrical power such as resulting from failure of an engine-mounted generator) with ozone provided to an ozone storage or distribution 34. ODA can be produced at the cathode 14 in each of these alternate modes of operation.

The term "about" is intended to include the degree of error associated with measurement of the particular quantity based upon the equipment available at the time of filing the application.

As used herein, the singular forms "a", "an", "the", or "any" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. In addition, many modifications may be made to adapt a particular situation or material to the teachings of the invention as defined by the claims without departing from the scope of the invention. Therefore, it is intended that the present disclosure not be limited to the particular embodiment disclosed as the best mode contemplated for carrying out this present disclosure, but that the present disclosure will include all embodiments falling within the scope of the claims.

## Claims

1. A vehicle (101) comprising an on-board inert gas-generating system, the system comprising:
a protected space (54) selected from a fuel tank ullage space, a cargo hold, or an equipment bay;
an electrochemical cell (10) comprising a cathode (14) and an anode (16) separated by a separator (12) comprising a proton transfer medium;
a cathode fluid flow path (23) in operative fluid communication with the cathode between a cathode fluid flow path inlet and a cathode fluid flow path outlet;
a cathode supply fluid flow path (22) between an air source (52) and the cathode fluid flow path inlet, and an inerting gas flow path (24) in operative fluid communication with the cathode fluid flow path outlet and the protected space;
an anode fluid flow path (25) in operative fluid communication with the anode between an anode fluid flow path inlet and an anode fluid flow path outlet;
an anode supply fluid flow path (22') between a water source (28) and the anode fluid flow path inlet, and an ozone flow path (26') in operative fluid communication with the anode fluid flow path outlet and an ozone storage or distribution system (34); and
an electrical connection (18) between a power source (20) and the electrochemical cell;
a hydrogen source in operative fluid communication with the anode fluid flow path inlet;
an electrical connection (18) between the electrochemical cell (10) and a power sink (21); and
a controller (36) configured to operate the system in alternate modes of operation selected from a plurality of modes including:
a first mode in which process water is directed to the anode fluid flow path inlet, electric power is directed from the power source to the electrochemical cell to provide a voltage difference between the anode and the cathode, and a gas comprising ozone is directed from the anode fluid flow path outlet to the ozone storage or distribution system, and
a second mode in which hydrogen is directed from the hydrogen source to the anode fluid flow path inlet, electric power is directed from the electrochemical cell to the power sink, and the ozone storage or distribution system is isolated from the anode fluid flow path outlet.

2. The vehicle of claim 1, wherein the ozone flow path includes a gas-liquid separator (27) that receives a mixture comprising process water, oxygen, and ozone from the anode fluid flow path outlet and outputs a gas comprising ozone to the ozone storage or distribution system.

3. The vehicle of claim 1, further comprising a biologically active surface or material, wherein the ozone storage or distribution system is in controllable operative fluid communication with the biologically active surface or material, and optionally wherein the biologically active surface or material includes a water storage tank, or a water distribution system, or a fuel storage tank, or a fuel distribution system.

4. The vehicle of claim 3, wherein the ozone storage or distribution system is in controllable operative fluid communication with a liquid space or a vapor space of a water storage or supply tank, and/or wherein the ozone storage or distribution system is in controllable operative fluid communication with a water supply flow path.

5. The vehicle of claim 4, further comprising a controller (36) configured direct a gas comprising ozone to the gas-liquid contactor in response to a flow of water on the water supply flow through the gas-liquid contactor.

6. The vehicle of claim 6, the controller is configured to operate in the first mode continuously or at intervals under normal operating conditions, and to operate in the second mode in response to a demand for emergency electrical power.

7. A method of treating a biologically active surface or material and inerting a protected space on-board the vehicle according to claim 1, comprising:
delivering water to the anode of the electrochemical cell;
applying a voltage difference between the anode and the cathode to electrolyze water at the anode to form a mixture comprising protons and ozone;
transferring the ozone to the ozone storage or distribution system, and transferring ozone from the ozone storage or distribution system to the biologically active surface or material;
delivering air to the cathode and transferring the protons across the separator to the cathode, and reducing oxygen at the cathode to generate oxygen-depleted air;
directing the oxygen-depleted air from the cathode of the electrochemical cell to the protected space.

8. The method of claim 7, comprising directing a fluid from the anode fluid flow path outlet to a gas-liquid separator, and directing the gas mixture comprising ozone from the cathode fluid flow path outlet and outputs a gas comprising ozone to the ozone storage or distribution system.

9. The method of claim 8, further comprising operating the electrochemical cell and directing the gas comprising ozone to the gas-liquid separator in response to a flow of water on the aircraft water supply flow through the gas-liquid separator.

10. The method of claim 7, 8, or 9, wherein the biologically active surface or material includes a water storage tank, or a water distribution system, or a fuel storage tank, or a fuel distribution system.

11. The method of claim 10, wherein the biologically active surface or material includes a water storage tank, and the method includes sparging the gas comprising ozone through a liquid space in the water storage tank, or wherein the biologically active surface or material includes a water distribution system, and the method includes contacting gas flowing through the water distribution system with a stream of the gas comprising ozone, or wherein the biologically active surface or material includes a fuel storage tank or a fuel distribution system, and the method includes inerting the fuel storage tank or fuel distribution system, and adding the gas comprising ozone to the fuel tank or fuel distribution system.

12. The method of claim 11, wherein inerting the fuel storage tank or distribution system includes adding an inert gas to the fuel tank or fuel distribution system.

13. The method of any of claims 7 to 12, further comprising:
operating in alternate modes of operation selected from a plurality of modes including:
a first mode in which process water is directed to the anode fluid flow path inlet, electric power is directed from the power source to the electrochemical cell to provide a voltage difference between the anode and the cathode, and a gas comprising ozone is directed from the anode fluid flow path outlet to the ozone storage or distribution system, and
a second mode in which hydrogen is directed from the hydrogen source to the anode fluid flow path inlet, electric power is directed from the electrochemical cell to the power sink, and the ozone storage or distribution system is isolated from the anode fluid flow path outlet.

## Patentansprüche

1. Fahrzeug (101), das ein Bord-Inertgaserzeugungssystem umfasst, wobei das System Folgendes umfasst:
einen geschützten Raum (54), der aus einem Kraftstoffbehälterleerraum, einem Laderaum oder einer Ausrüstungskammer ausgewählt ist;
eine elektrochemische Zelle (10), die eine Kathode (14) und eine Anode (16) umfasst, die durch einen Separator (12) getrennt sind, der ein Protonentransfermedium umfasst;
einen Kathodenfluidströmungsweg (23) in wirksamer Fluidverbindung mit der Kathode zwischen einem Einlass des Kathodenfluidströmungswegs und einem Auslass des Kathodenfluidströmungswegs;
einen Kathodenzufuhrfluidströmungsweg (22) zwischen einer Luftquelle (52) und dem Einlass des Kathodenfluidströmungswegs und einen Inertgasströmungsweg (24) in wirksamer Fluidverbindung mit dem Auslass des Kathodenfluidströmungswegs und dem geschützten Raum;
einen Anodenfluidströmungsweg (25) in wirksamer Fluidverbindung mit der Anode zwischen einem Einlass des Anodenfluidströmungswegs und einem Auslass des Anodenfluidströmungswegs;
einen Anodenzufuhrfluidströmungsweg (22') zwischen einer Wasserquelle (28) und dem Einlass des Anodenfluidströmungswegs und einen Ozonströmungsweg (26') in wirksamer Fluidverbindung mit dem Auslass des Anodenfluidströmungswegs und einem Ozonspeicher- oder -verteilungssystem (34); und
eine elektrische Verbindung (18) zwischen einer Stromquelle (20) und der elektrochemischen Zelle;
eine Wasserstoffquelle in wirksamer Fluidverbindung mit dem Einlass des Anodenfluidströmungswegs;
eine elektrische Verbindung (18) zwischen der elektrochemischen Zelle (10) und einer Stromsenke (21); und
eine Steuerung (36), die so konfiguriert ist, dass sie das System in alternierenden Betriebsmodi betreibt, die aus einer Vielzahl von Modi ausgewählt sind, die Folgendes beinhalten:
einen ersten Modus, in dem Prozesswasser zum Einlass des Anodenfluidströmungswegs geleitet wird, elektrischer Strom von der Stromquelle zur elektrochemischen Zelle geleitet wird, um eine Spannungsdifferenz zwischen der Anode und der Kathode bereitzustellen, und ein Gas, das Ozon umfasst, von dem Auslass des Anodenfluidströmungswegs zum Ozonspeicher- oder - verteilungssystem geleitet wird, und
einen zweiten Modus, in dem Wasserstoff von der Wasserstoffquelle zum Einlass des Anodenfluidströmungswegs geleitet wird, elektrischer Strom von der elektrochemischen Zelle zur Stromsenke geleitet wird und das Ozonspeicher- oder -verteilungssystem von dem Auslass des Anodenfluidströmungswegs isoliert ist.

2. Fahrzeug nach Anspruch 1, wobei der Ozonströmungsweg einen Gas-Flüssigkeits-Separator (27) beinhaltet, der ein Gemisch, das Prozesswasser, Sauerstoff und Ozon umfasst, aus dem Auslass des Anodenfluidströmungswegs aufnimmt, und ein Gas, das Ozon umfasst, an das Ozonspeicher- oder -verteilungssystem ausgibt.

3. Fahrzeug nach Anspruch 1, ferner eine biologisch aktive Oberfläche oder ein biologisch aktives Material umfassend, wobei das Ozonspeicher- oder -verteilungssystem in steuerbarer wirksamer Fluidverbindung mit der biologisch aktiven Oberfläche oder dem biologisch aktiven Material steht, und optional wobei die biologisch aktive Oberfläche oder das biologisch aktive Material einen Wasserspeicherbehälter oder ein Wasserverteilungssystem oder einen Kraftstoffspeicherbehälter oder ein Kraftstoffverteilungssystem beinhaltet.

4. Fahrzeug nach Anspruch 3, wobei das Ozonspeicher- oder -verteilungssystem in steuerbarer wirksamer Fluidverbindung mit einem Flüssigkeitsraum oder einem Dampfraum eines Wasserspeicher- oder -zufuhrbehälters steht und/oder wobei das Ozonspeicher- oder -verteilungssystem in steuerbarer wirksamer Fluidverbindung mit einem Wasserzufuhrströmungsweg steht.

5. Fahrzeug nach Anspruch 4, ferner eine Steuerung (36) umfassend, die so konfiguriert ist, dass sie ein Gas, das Ozon umfasst, an den Gas-Flüssigkeits-Kontaktor als Reaktion auf einen Strom von Wasser an der Wasserzufuhrströmung durch den Gas-Flüssigkeits-Kontaktor leitet.

6. Fahrzeug nach Anspruch 6, wobei die Steuerung so konfiguriert ist, dass sie unter normalen Betriebsbedingungen durchgängig oder in Intervallen in dem ersten Modus arbeitet und als Reaktion auf einen Bedarf an elektrischem Notstrom in dem zweiten Modus arbeitet.

7. Verfahren zum Behandeln einer biologisch aktiven Fläche oder eines biologisch aktiven Materials und Inertisieren eines geschützten Raums an Bord des Fahrzeugs nach Anspruch 1, umfassend:
Liefern von Wasser zur Anode der elektrochemischen Zelle;
Anlegen einer Spannungsdifferenz zwischen der Anode und der Kathode, um Wasser an der Anode zu elektrolysieren, um ein Gemisch zu bilden, das Protonen und Ozon umfasst;
Überführen des Ozons zum Ozonspeicher- oder
- verteilungssystem und Überführen des Ozons vom Ozonspeicher- oder
- verteilungssystem zur biologisch aktiven Fläche oder zum biologisch aktiven Material;
Liefern von Luft zur Kathode und Überführen der Protonen über den Separator zur Kathode und Verringern des Sauerstoffs an der Kathode, um sauerstoffarme Luft zu erzeugen;
Leiten der sauerstoffarmen Luft von der Kathode der elektrochemischen Zelle zum geschützten Raum.

8. Verfahren nach Anspruch 7, das Leiten eines Fluids vom Auslass des Anodenfluidströmungswegs zu einem Gas-Flüssigkeits-Separator und das Leiten des Gasgemischs, das Ozon umfasst, von dem Auslass des Kathodenfluidströmungswegs umfassend, und gibt ein Gas, das Ozon umfasst, an das Ozonspeicher- oder - verteilungssystem aus.

9. Verfahren nach Anspruch 8, ferner das Betreiben der elektrochemischen Zelle und das Leiten des Gases, das Ozon umfasst, an den Gas-Flüssigkeits-Separator als Reaktion auf einen Strom von Wasser an der Luftfahrzeugwasserzufuhrströmung durch den Gas-Flüssigkeits-Separator umfassend.

10. Verfahren nach Anspruch 7, 8, oder 9, wobei die biologisch aktive Oberfläche oder das biologisch aktive Material einen Wasserspeicherbehälter oder ein Wasserverteilungssystem oder einen Kraftstoffspeicherbehälter oder ein Kraftstoffverteilungssystem beinhaltet.

11. Verfahren nach Anspruch 10, wobei die biologisch aktive Oberfläche oder das biologisch aktive Material einen Wasserspeicherbehälter beinhaltet und das Verfahren das Durchperlenlassen des Gases, das Ozon umfasst, durch einen Flüssigkeitsraum in dem Wasserspeicherbehälter beinhaltet oder wobei die biologisch aktive Oberfläche oder das biologisch aktive Material ein Wasserverteilungssystem beinhaltet und das Verfahren das Inkontaktbringen des Gases, das durch das Wasserverteilungssystem strömt, mit einem Strom des Gases, das Ozon umfasst, beinhaltet oder wobei die biologisch aktive Oberfläche oder das biologisch aktive Material einen Kraftstoffspeicherbehälter oder ein Kraftstoffverteilungssystem beinhaltet und das Verfahren das Inertisieren des Kraftstoffspeicherbehälters oder des Kraftstoffverteilungssystems und das Zufügen des Gases, das Ozon umfasst, zu dem Kraftstoffspeicherbehälter oder dem Kraftstoffverteilungssystem beinhaltet.

12. Verfahren nach Anspruch 11, wobei das Inertisieren des Kraftstoffspeicherbehälters oder -verteilungssystems das Zugeben eines Inertgases zu dem Kraftstoffspeicherbehälter oder dem Kraftstoffverteilungssystem beinhaltet.

13. Verfahren nach einem der Ansprüche 7 bis 12, ferner Folgendes umfassend:
Betreiben in alternierenden Betriebsmodi, die aus einer Vielzahl von Modi ausgewählt sind, die Folgendes beinhalten:
einen ersten Modus, in dem Prozesswasser zum Einlass des Anodenfluidströmungswegs geleitet wird, elektrischer Strom von der Stromquelle zur elektrochemischen Zelle geleitet wird, um eine Spannungsdifferenz zwischen der Anode und der Kathode bereitzustellen, und ein Gas, das Ozon umfasst, von dem Auslass des Anodenfluidströmungswegs zum Ozonspeicher- oder - verteilungssystem geleitet wird, und
einen zweiten Modus, in dem Wasserstoff von der Wasserstoffquelle zum Einlass des Anodenfluidströmungswegs geleitet wird, elektrischer Strom von der elektrochemischen Zelle zur Stromsenke geleitet wird und das Ozonspeicher- oder - verteilungssystem von dem Auslass des Anodenfluidströmungswegs isoliert ist.

## Revendications

1. Véhicule (101) comprenant un système embarqué de génération de gaz inerte, le système comprenant :
un espace protégé (54) choisi parmi un espace mort de réservoir de carburant, une soute, ou un compartiment équipement ;
une cellule électrochimique (10) comprenant une cathode (14) et une anode (16) séparées par un séparateur (12) comprenant un milieu de transfert de protons ;
un trajet d'écoulement de fluide de cathode (23) en communication fluidique opérationnelle avec la cathode entre une entrée de trajet d'écoulement de fluide de cathode et une sortie de trajet d'écoulement de fluide de cathode ;
un trajet d'écoulement de fluide d'alimentation de cathode (22) entre une source d'air (52) et l'entrée de trajet d'écoulement de fluide de cathode, et un trajet d'écoulement de gaz d'inertage (24) en communication fluidique opérationnelle avec la sortie de trajet d'écoulement de fluide de cathode et l'espace protégé ;
un trajet d'écoulement de fluide d'anode (25) en communication fluidique opérationnelle avec l'anode entre une entrée de trajet d'écoulement de fluide d'anode et une sortie de trajet d'écoulement de fluide d'anode ;
un trajet d'écoulement de fluide d'alimentation d'anode (22') entre une source d'eau (28) et l'entrée de trajet d'écoulement de fluide d'anode, et un trajet d'écoulement d'ozone (26') en communication fluidique opérationnelle avec la sortie de trajet d'écoulement de fluide d'anode et un système de stockage ou de distribution d'ozone (34) ; et
une liaison électrique (18) entre une source d'alimentation (20) et la cellule électrochimique ;
une source d'hydrogène en communication fluidique opérationnelle avec l'entrée de trajet d'écoulement de fluide d'anode ;
une liaison électrique (18) entre la cellule électrochimique (10) et un récepteur d'énergie (21) ; et
un dispositif de commande (36) conçu pour faire fonctionner le système dans des modes de fonctionnement alternatifs choisis parmi une pluralité de modes comportant :
un premier mode dans lequel l'eau de traitement est dirigée vers l'entrée de trajet d'écoulement de fluide d'anode, l'énergie électrique est dirigée de la source d'alimentation vers la cellule électrochimique pour fournir une différence de tension entre l'anode et la cathode, et un gaz comprenant de l'ozone est dirigé de la sortie de trajet d'écoulement de fluide d'anode vers le système de stockage ou de distribution d'ozone, et
un second mode dans lequel l'hydrogène est dirigé de la source d'hydrogène vers l'entrée de trajet d'écoulement de fluide d'anode, l'énergie électrique est dirigée de la cellule électrochimique vers le récepteur d'énergie, et le système de stockage ou de distribution d'ozone est isolé de la sortie de trajet d'écoulement de fluide d'anode.

2. Véhicule selon la revendication 1, dans lequel le trajet d'écoulement d'ozone comporte un séparateur gaz-liquide (27) qui reçoit un mélange comprenant l'eau de traitement, de l'oxygène et de l'ozone provenant de la sortie de trajet d'écoulement de fluide d'anode et délivre en sortie un gaz comprenant de l'ozone au système de stockage ou de distribution d'ozone.

3. Véhicule selon la revendication 1, comprenant en outre une surface ou un matériau biologiquement actif, dans lequel le système de stockage ou de distribution d'ozone est en communication fluidique opérationnelle réglable avec la surface ou le matériau biologiquement actif, et éventuellement dans lequel la surface ou le matériau biologiquement actif comporte un réservoir de stockage d'eau, ou un système de distribution d'eau, ou un réservoir de stockage de carburant, ou un système de distribution de carburant.

4. Véhicule selon la revendication 3, dans lequel le système de stockage ou de distribution d'ozone est en communication fluidique opérationnelle réglable avec un espace de liquide ou un espace de vapeur d'un réservoir de stockage ou d'alimentation en eau, et/ou dans lequel le système de stockage ou de distribution d'ozone est en communication fluidique opérationnelle réglable avec un trajet d'écoulement d'alimentation en eau.

5. Véhicule selon la revendication 4, comprenant en outre un dispositif de commande (36) conçu pour diriger un gaz comprenant de l'ozone vers le contacteur gaz-liquide en réponse à un écoulement d'eau sur l'écoulement d'alimentation en eau à travers le contacteur gaz-liquide.

6. Véhicule selon la revendication 6, dans lequel le dispositif de commande est conçu pour fonctionner dans le premier mode en continu ou à intervalles dans des conditions de fonctionnement normales, et pour fonctionner dans le second mode en réponse à une demande d'alimentation électrique d'urgence.

7. Procédé de traitement d'une surface ou d'un matériau biologiquement actif et d'inertage d'un espace protégé à bord du véhicule selon la revendication 1, comprenant :
la fourniture d'eau à l'anode de la cellule électrochimique ;
l'application d'une différence de tension entre l'anode et la cathode pour électrolyser l'eau au niveau de l'anode afin de former un mélange comprenant des protons et de l'ozone ;
le transfert de l'ozone vers le système de stockage ou de distribution d'ozone, et le transfert d'ozone du système de stockage ou de distribution d'ozone vers la surface ou le matériau biologiquement actif ;
la fourniture d'air à la cathode et le transfert des protons à travers le séparateur vers la cathode, et la réduction de l'oxygène au niveau de la cathode pour générer de l'air appauvri en oxygène ;
le fait de diriger l'air appauvri en oxygène de la cathode de la cellule électrochimique vers l'espace protégé.

8. Procédé selon la revendication 7, comprenant le fait de diriger un fluide de la sortie de trajet d'écoulement de fluide d'anode vers un séparateur gaz-liquide, et de diriger le mélange gazeux comprenant de l'ozone depuis la sortie de trajet d'écoulement de fluide de cathode et la livraison en sortie d'un gaz comprenant de l'ozone vers le système de stockage ou de distribution d'ozone.

9. Procédé selon la revendication 8, comprenant en outre le fait de faire fonctionner la cellule électrochimique et de diriger le gaz comprenant de l'ozone vers le séparateur gaz-liquide en réponse à un écoulement d'eau sur l'écoulement d'alimentation en eau d'aéronef à travers le séparateur gaz-liquide.

10. Procédé selon la revendication 7, 8 ou 9, dans lequel la surface ou le matériau biologiquement actif comporte un réservoir de stockage d'eau, ou un système de distribution d'eau, ou un réservoir de stockage de carburant, ou un système de distribution de carburant.

11. Procédé selon la revendication 10, dans lequel la surface ou le matériau biologiquement actif comporte un réservoir de stockage d'eau, et le procédé comporte le barbotage du gaz comprenant de l'ozone à travers un espace de liquide dans le réservoir de stockage d'eau, ou dans lequel la surface ou le matériau biologiquement actif comporte un système de distribution d'eau, et le procédé comporte la mise en contact d'un gaz s'écoulant à travers le système de distribution d'eau avec un courant du gaz comprenant de l'ozone, ou dans lequel la surface ou le matériau biologiquement actif comporte un réservoir de stockage de carburant ou un système de distribution de carburant, et le procédé comporte l'inertage du réservoir de stockage de carburant ou du système de distribution de carburant, et l'ajout du gaz comprenant de l'ozone au réservoir de carburant ou au système de distribution de carburant.

12. Procédé selon la revendication 11, dans lequel l'inertage du réservoir de stockage de carburant ou du système de distribution de carburant comporte l'ajout d'un gaz inerte au réservoir de carburant ou au système de distribution de carburant.

13. Procédé selon l'une quelconque des revendications 7 à 12, comprenant en outre :
le fonctionnement dans des modes de fonctionnement alternés choisis parmi une pluralité de modes comportant :
un premier mode dans lequel l'eau de traitement est dirigée vers l'entrée de trajet d'écoulement de fluide d'anode, l'énergie électrique est dirigée de la source d'alimentation vers la cellule électrochimique pour fournir une différence de tension entre l'anode et la cathode, et un gaz comprenant de l'ozone est dirigé de la sortie de trajet d'écoulement de fluide d'anode vers le système de stockage ou de distribution d'ozone, et
un second mode dans lequel l'hydrogène est dirigé de la source d'hydrogène vers l'entrée de trajet d'écoulement de fluide d'anode, l'énergie électrique est dirigée de la cellule électrochimique vers le récepteur d'énergie, et le système de stockage ou de distribution d'ozone est isolé de la sortie de trajet d'écoulement de fluide d'anode.
